# EUROPÄISCHE PATENTSCHRIFT

(11) **EP 0 607 468 B1**
(45) Veröffentlichungstag und Bekanntmachung des Hinweises auf die Patenterteilung: **21.09.1994**
(21) Anmeldenummer: 92811008.9
(22) Anmeldetag: 16.12.1992
(51) Int. Cl.: A61F 2/06, A61M 25/04

(54) **Vorrichtung zum Implantieren einer selbstexpandierenden Endoprothese**
Stent placement instrument
Dispositif d'introduction d'un dilatateur

(43) Veröffentlichungstag der Anmeldung: 27.07.1994
(73) Patentinhaber: SCHNEIDER (EUROPE) AG, 8180 Bülach (CH)
(72) Erfinder: Hofmann, Eugen, CH-8064 Zürich (CH); Gianotti, Marc, CH-8542 Wiesendangen (CH)
(74) Vertreter: Groner, Manfred

(56) Entgegenhaltungen:
- EP-A- 0 408 245
- US-A- 4 665 918

## Beschreibung

Die Erfindung betrifft eine Vorrichtung zum Implantieren einer selbstexpandierenden Endoprothese in ein Gefäss, mit einem flexiblen länglichen Aussenkatheter mit einem distalen Ende und einem proximalen Ende und mit einem koaxial zum Aussenkatheter angeordneten flexiblen länglichen Innenkatheter, der an einem distalen Ende eine Spitze und proximal dieser am Umfang eine Vertiefung zur Aufnahme der Endoprothese aufweist, wobei zur Fixierung der Endoprothese der Aussenkatheter in seiner Längsrichtung über die Vertiefung schiebbar und zur Freigabe der Endoprothese in das Gefäss zurückziehbar ist.

Eine solche Vorrichtung ist z.B. aus der US-A-4 665 918 bekannt.

Für die Behandlung von arteriellen Verschlusskrankheiten werden seit einiger Zeit selbstexpandierende Endoprothesen verwendet, die mittels einer Vorrichtung der genannten Gattung in das zu behandelnde Gefäss eingeführt werden. Eine bekannte Endoprothese ist die "Wallstent^{R}-Endoprothese", die aus einem rohrförmigen Drahtgewebe aus Stahl besteht. Endoprothesen dieser Art werden häufig als "Stent" bezeichnet.

Eine bekannte Vorrichtung zum Implantieren solcher Endoprothesen wird von der Anmelderin hergestellt. Der Innenkatheter dieser Vorrichtung weist ein flexibles Rohr auf, das unmittelbar hinter der distalen Spitze aussenseitig eine eingearbeitete Vertiefung aufweist. In diese Vertiefung wird die in das Gefäss einzuführende Endoprothese eingeschoben. Beim Zurückziehen des Aussenkatheters liegt die Endoprothese proximal an einer Schulter dieser Vertiefung an. Die Vertiefung dient somit zur Aufnahme der Endoprothese und verhindert beim Zurückziehen des Aussenkatheters dass diese axial auf dem Innenkatheter verschoben wird. Durch den Aussenkatheter wird die Endoprothese in einem vorgespannten Zustand fixiert. Durch einen Rückzug des Aussenkatheters wird die Endoprothese freigegeben, die sich hierbei auf Kosten ihrer Länge auf einen vorgegebenen Aussendurchmesser ausdehnt. Die Endoprothese kann sich dadurch innenseitig an die zu behandelnde Gefässwand anlegen. Nach dem Freisetzen der Endoprothese wird die Vorrichtung aus dem Gefäss zurückgezogen. In der Praxis hat sich die Handhabung dieser Vorrichtung als einfach, rasch und sicher erwiesen. Aehnliche Katheter sind durch die US-A-5,026,377, die EP-A-0 416 662 und durch die EP-A-0 418 677 bekanntgeworden.

Zunehmend werden auch Endoprothesen mit vergleichsweise grossem Aussendurchmesser implantiert. Damit diese Endoprothesen den erforderlichen Stützdruck auf das Gefäss ausüben können, weisen diese entsprechend eine grössere Anzahl Drähte auf. Gleichzeitig sind diese Drähte auch dicker als bei kleineren Endoprothesen üblich. Zum Implantieren dieser vergleichsweise grossen Endoprothesen mussten bisher Vorrichtungen mit einem entsprechend grösseren Aussendurchmesser verwendet werden. Die Vertiefung am Innenkatheter kann nämlich nicht beliebig weit vertieft werden um eine umfangreichere Endoprothese aufzunehmen. Wenn die Vertiefung zu stark ausgeprägt ist, würde der Innenkatheter in gewundenen Gefässstrecken im Bereich der Vertiefung knicken. Ein grösserer Aussendurchmesser bedeutete bisher einerseits eine grössere Punktionsstelle und andererseits eine geringere Zugänglichkeit zu engeren Gefässen und auch eine schwierigere Steuerbarkeit, beispielsweise beim Einführen der Vorrichtung in einen Seitenast eines Gefässes. Eine Vorrichtung mit einem grösseren Aussendurchmesser belastet einen Patienten wesentlich stärker als eine solche mit einem kleineren Aussendurchmesser. Einerseits ist die Punktionsstelle bei grösserem Aussendurchmesser der Vorrichtung entsprechend grösser. Entsprechend wird die Gefahr von Komplikationen nach Entfernen der Vorrichtung grösser. Eine schlechtere Zugänglichkeit bedeutet eine längere Behandlungsdauer und damit ebenfalls eine höhere Belastung des Patienten. Eine höhere Belastung des Patienten tritt auch deshalb auf, weil eine Vorrichtung mit grösserem Aussendurchmesser den Fluss des Gefässmediums auf der Länge in der sich die Vorrichtung im Gefäss befindet stärker behindert. Wünschbar wäre somit eine gattungsgemässe Vorrichtung, die bei gleichen Endoprothesen einen kleineren Aussendurchmesser aufweisen. Die Vorrichtung soll trotzdem eine einfache, rasche und sichere Handhabung gewährleisten und ohne grossen Aufwand herstellbar sein.

Die Aufgabe ist bei einer gattungsgemässen Vorrichtung dadurch gelöst, dass die Vertiefung zur Aufnahme der Endoprothese durch ein separates Zwischenstück des Innenkatheters gebildet ist.

Bei der erfindungsgemässen Vorrichtung besteht bezüglich des Zwischenstückes weitgehende Freiheit in der Materialwahl, den Abmessungen und der Herstellungsmethode. So sind beispielsweise an das Zwischenstück anders als an den restlichen Innenkatheterschaft keine besonderen Anforderungen gestellt bezüglich Gleiteigenschaften und Schubübertragung. Das Zwischenstück kann daher in seinem Material, seinen Abmessungen und in seiner Herstellmethode unabhängig von diesen Eigenschaften gestaltet werden. Der Stammabschnitt des Innenkatheterschaftes kann demgegenüber in seinem Material, seinen Abmessungen und seiner Herstellmethode so ausgewählt werden, dass diese Eigenschaften besonders ausgeprägt sind. Im Bereich der Vertiefung kann der Innenkatheter bei mindestens gleicher Flexibilität und Knickfestigkeit mit einem geringeren Aussendurchmesser ausgebildet werden. Ausserhalb der Vertiefung kann der Innenkatheter beispielsweise ohne Rücksicht auf die Abmessung der Vertiefung zur Erzielung einer grösseren Flexibilität des Katheterschaftes einen grösseren Innendurchmesser aufweisen. Ein geringerer Aussendurchmesser im Bereich der Vertiefung bedeutet eine Vergrösserung des Volumens für die Aufnahme der Endoprothese. Bei gleichem Innendurchmesser des Aussenkatheters und bei gleicher axialer Erstreckung der Vertiefung steht bei der erfindungsgemässen Vorrichtung mehr Raum zur Unterbringung der Endoprothese zur Verfügung. Bei gleichem Innendurchmesser des Aussenkatheters kann somit eine grössere Endoprothese montiert werden. Ohne die Belastung für den Patienten zu erhöhen, stehen somit mehr und bessere Behandlungsmethoden zur Verfügung, z.B. Behandlung mit grösseren Endoprothesen, Behandlung mit besser verträglichen Endoprothesen, deren Einzeldrähte oder Fasern z.B. beschichtet sind, Behandlung mit Endoprothesen mit stärkerer Expansionskraft oder Behandlung mit Endoprothesen, die durch eine schlauchförmige Hülle das Einwachsen von Tumoren in das Gefäss verhindern sollen. Die Vorrichtung ist auch ohne grossen Aufwand herstellbar. Der zur Erzielung der notwendigen Eigenschaften im Bereich der Vertiefung notwendige hohe Herstellaufwand erstreckt sich nicht auf die gesamte Vorrichtungslänge, sondern bleibt im wesentlichen auf die Länge der Vertiefung beschränkt. Für die restliche Innenkatheterlänge kann dann relativ einfaches Rohmaterial benutzt werden.

Es hat sich nun überraschend gezeigt, dass die erfindungsgemässe Vorrichtung auch bezüglich des Montierens der Endoprothese wesentlich einfacher zu handhaben ist als die vorbekannte Vorrichtung. Da wie oben erwähnt bei gleichem Aussendurchmesser mehr Raum zur Unterbringung der Endoprothese zur Verfügung steht, ist es auch wesentlich einfacher, die Endoprothese zu montieren. War es bisher notwendig, zum Montieren der Endoprothese eine spezielle Vorrichtung zu verwenden, so ist es nun bei der erfindungsgemässen Vorrichtung möglich, die Endoprothese von Hand zu montieren. In der Praxis bedeutet dies, dass die Endoprothese nicht wie bisher vormontiert werden muss, sondern unmittelbar vor der Verwendung der Vorrichtung montiert werden kann. Hierbei kann die Endoprothese in der gewünschten Länge von einem längeren Stück abgeschnitten werden. Einerseits wird vermieden, dass die Endoprothese in vorgespanntem Zustand sterilisiert werden muss und durch lange Lagerung im vorgespannten Zustand in der Vorrichtung an Spannung verliert und andererseits wird vermieden, dass beim Arzt eine grössere Anzahl Vorrichtungen montiert, mit unterschiedlich langen Endoprothesen gelagert und zur Verfügung gehalten werden muss.

Nach einer Weiterbildung ist das Zwischenstück verstärkt. Dies kann beispielsweise durch eine geeignete Formgebung, beispielsweise mittels Rippen auf der Aussenseite oder beispielsweise durch eine Art Armierung des Zwischenstückes erfolgen. Besonders geeignet ist ein Zwischenstück aus einem Verbundwerkstoff. Geeignet sind ebenfalls Zwischenstücke die mittels Fasern, insbesondere Glasfasern verstärkt sind oder die wenigstens teilweise aus Metall hergestellt sind.

In der Regel genügt es, wenn das Zwischenstück im wesentlichen die Länge der Vertiefung aufweist. Das Zwischenstück kann somit vergleichsweise kurz sein, so dass auch teure Werkstoffe zur Herstellung des Zwischenstücks die Herstellungskosten für die Vorrichtung nicht wesentlich beeinflussen. Weitere vorteilhafte Merkmale ergeben sich aus den abhängigen Ansprüchen, der nachfolgenden Beschreibung sowie der Zeichnung.

Ausführungsbeispiele der Erfindung werden nachfolgend anhand der Zeichnung erläutert. Es zeigen:
- Fig. 1: einen Schnitt durch das distale Ende einer erfindungsgemässen Vorrichtung,
- Fig. 2: das distale Ende gemäss Fig. 1, eingeführt in ein Gefäss und mit teilweise freigesetzter Endoprothese,
- Fig. 3: das distale Ende einer erfindungsgemässen Variante der Vorrichtung, ebenfalls im Längsschnitt, und
- Fig. 4: eine teilweise geschnittene Ansicht einer erfindungsgemässen Vorrichtung.

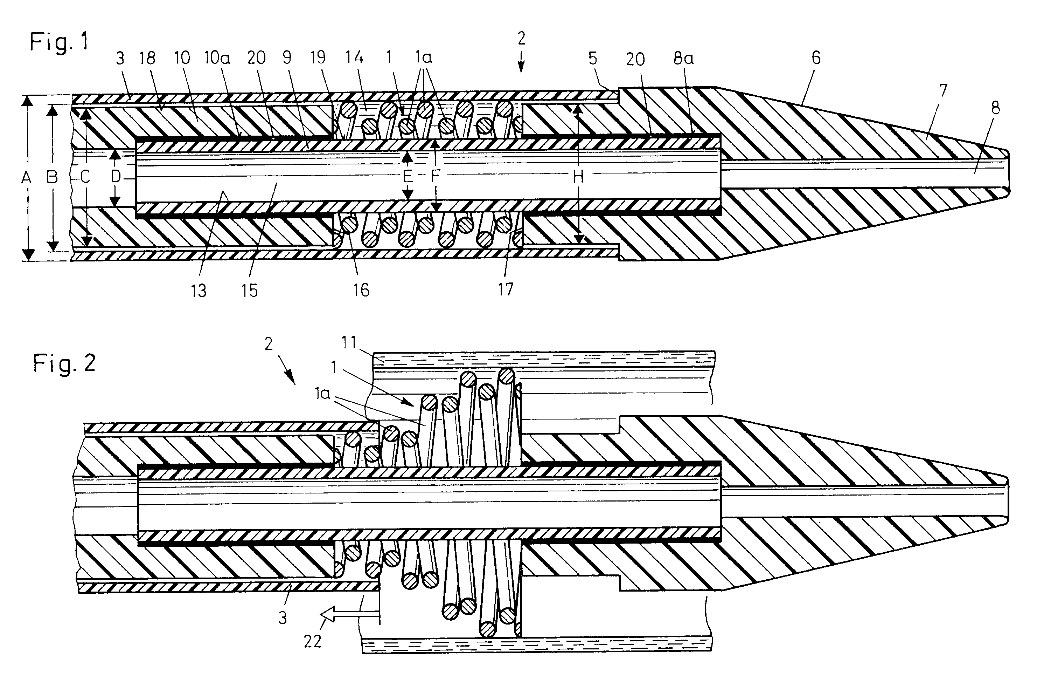

Die Fig. 1 zeigt eine Endoprothese 1, die in einer erfindungsgemässen Vorrichtung 2 montiert ist. Die Endoprothese 1 ist aus rostfreien Stahldrähten 1a hergestellt und kann aussenseitig mit einer hier nicht gezeigten dehnbaren Hülle versehen sein. Ueber die radial gespannte Endoprothese 1 ist ein Aussenkatheter 3 geschoben, der im wesentlichen ein flexibles längliches Rohr mit einer distalen Mündung 5 ist. Die Endoprothese 1 liegt innenseitig am Aussenkatheter an. Der Aussenkatheter 3 verhindert eine radiale Expansion der Endoprothese 1. Sowohl die Endoprothese 1 als auch der Aussenkatheter 3 sind dem Fachmann an sich bekannt.

Ein Innenkatheter 6 ist von der Mündung 5 her in den Aussenkatheter 3 eingeführt. Dieser ebenfalls längliche und flexible Innenkatheter 6 kann ein durchgehendes Lumen 15 zur Aufnahme eines an sich bekannten Führungsdrahtes aufweisen. Möglich ist jedoch auch eine Ausführung ohne ein inneres Lumen 15. In diesem Fall kann am distalen Ende des Innenkatheters 6 ein hier nicht gezeigter Führungsdraht fest angebracht sein. Wie aus Fig. 1 ersichtlich, weist der Innenkatheter 6 eine Spitze 7, ein Zwischenstück 9 sowie ein proximal zum Zwischenstück 9 angeordnetes Rohrstück 10 auf. Das Zwischenstück 9 ist aussenseitig zylindrisch und weist einen Aussendurchmesser F auf, der kleiner ist als ein Aussendurchmesser C des Rohrstücks 10 und auch kleiner als der Aussendurchmesser H der Spitze 7. Die Aussendurchmesser C und H sind im wesentlichen gleich und nur geringfügig kleiner als ein Durchmesser B einer zylindrischen Innenseite 18 des Aussenkatheters 3. Im Abstand zueinander angeordnete Querschnittsflächen 16 und 17 des Rohrstücks 10 und der Spitze 7 sowie die Innenseite 18 des Aussenkatheters 3 und eine zylindrische Aussenseite 19 des Zwischenstücks 9 bilden einen hohlzylindrischen Raum 14 in dem die montierte Endoprothese 1 untergebracht ist. Der Abstand der Flächen 16 und 17 ist im wesentlichen durch die Länge der Endoprothese 1 im montierten Zustand gegeben.

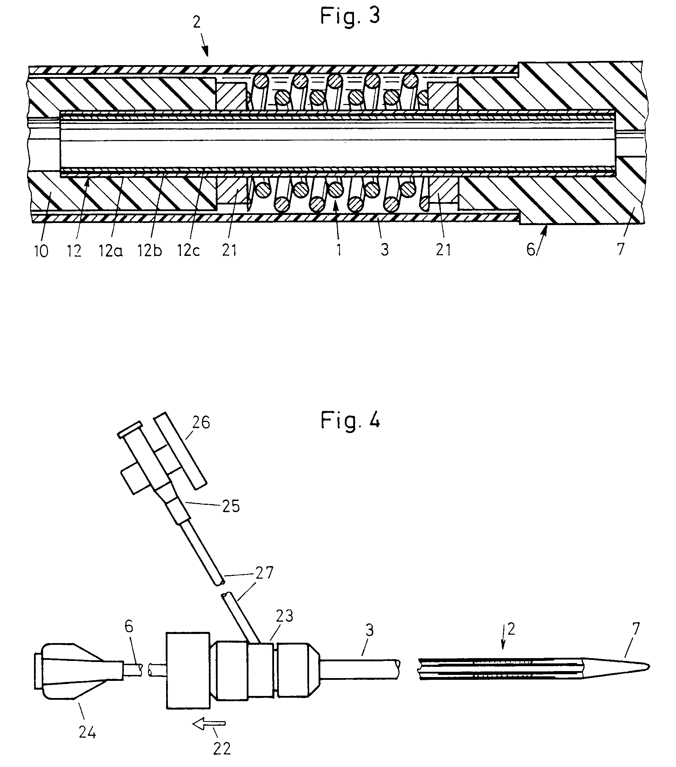

Wie ersichtlich, ist das Zwischenstück 9 etwas länger als der Abstand zwischen den Flächen 16 und 17 und mit dem einen Ende in eine erweiterte Ausnehmung 10a des Rohrstücks 10 bzw. eine erweiterte Ausnehmung 8a der Spitze 7 eingesetzt. Diese Enden sind beispielsweise mit einem geeigneten Klebstoff 20 mit dem Rohrstück 10 bzw. der Spitze 7 fest verbunden. Denkbar sind hier auch andere Verbindungen, beispielsweise durch Verschweissen des Zwischenstücks 9 mit dem Rohrstück 10 bzw. der Spitze 7. Das Zwischenstück 9 kann aus einem Material hergestellt sein, das verglichen mit den Werkstoff des Rohrstücks 10 bei kleinerem Aussendurchmesser F ebenso flexibel und knickfest ist. Dazu eignet sich beispielweise ein verstärkter Werkstoff. Beispielsweise kann dazu das Zwischenstück 9 aus einem Verbundwerkstoff hergestellt sein. Die Fig. 3 zeigt hierzu ein Zwischenstück 12, das aus koaxialen Schichten 12a, 12b und 12c hergestellt ist. Solche Verbundwerkstoffe sind an sich bekannt. Das Zwischenstück 9 kann mittels Fasern, insbesondere Glasfasern verstärkt sein. Denkbar ist auch eine Ausführung, bei der das Zwischenstück 9 auch mit einer Metalleinlage verstärkt ist. Ein solches Zwischenstück ist bei vergleichsweise kleinem Aussendurchmesser F gleichzeitig flexibel und knickfest, wie dies für den Innenkatheter 6 insbesondere im distalen Bereich besonders dann erforderlich ist, wenn die Endoprothese 1 in einem gebogenen Bereich eines Gefässes eingeführt werden soll. Selbstverständlich ist auch der Aussenkatheter 3 und damit die gesamte Vorrichtung 2 im wesentlichen auf ihrer ganzen Länge flexibel. Die genannten Verstärkungen des Zwischenstücks 9 bzw. 12 gewährleisten die erforderliche Knickfestigkeit des Innenkatheters 6 im Bereich der Endoprothese 1.

Eine für die Einführung einer Endoprothese 1 mit bis zu 40 mm entspanntem Aussendurchmesser geeignete Vorrichtung 2 weist beispielsweise die folgenden Masse auf: A = 5,2 mm, B = 4,6 mm, C = 4,5 mm, D = 1,8 mm, F = 2,00 mm, E = 1,6 mm und H = 4,53 mm. Der Unterschied zwischen den Durchmessern der Flächen 18 und 19 beträgt somit 2,25 mm. Dieser Unterschied ist hinreichend gross, um eine umhüllte Endoprothese mit bis zu 25 mm entspanntem Aussendurchmesser oder eine unbeschichtete Endoprothese mit bis zu 40 mm entspanntem Aussendurchmesser zu montieren. Der Aussenkatheter 3 ist hierbei aus Tetraflouräthylen und das Rohrstück 10 sowie die Spitze 7 aus Polyurethan hergestellt. Auf das Zwischenstück 9 sind in der Regel Markierringe 21 aus Gold oder Tantal aufgesetzt. Solche Markierringe sind in der Fig. 3 gezeigt. Wie die Fig. 3 zeigt, können die Ringe 21 Schultern bilden, an denen die montierte Endoprothese anliegt. Anhand der Fig. 2 und 4 soll nachfolgend kurz die Verwendung der Vorrichtung 2 erläutert werden.

Das Bezugszeichen 11 bezeichnet eine Gefässwandung beispielweise eines Blutgefässes, das vorgängig beispielsweise mit einem Balloonkatheter erweitert worden ist. Um diese Aufweitung mit einer Endoprothese 1 zu stabilisieren, wird das distale Ende der Vorrichtung 2 über eine hier nicht gezeigte an sich bekannte Einführungsschleuse in das Gefäss eingeführt, bis die Endoprothese 1 an den geeigneten Ort vorgeschoben ist. Hierbei ist die Endoprothese 1 durch den Aussenkatheter 3 fixiert. Durch Rückzug des Aussenkatheters 3 an einem fest mit diesem verbundenen Anschluss- und Dichtungsstück 23 in Richtung des Pfeils 22 wird die Endoprothese 1 allmählich freigegeben, wie dies in Fig. 2 gezeigt ist. Der Innenkatheter 6 wird hierbei gleichzeitig an seinem proximalen Ende, das ein Verbindungsstück 24 zum Einspritzen von Kontrastmittel und Einführen eines Führungsdrahtes aufweist, festgehalten. Die Endoprothese 1 ist freigesetzt, sobald die Mündung 5 des Aussenkatheters 3 über die Fläche 16 des Rohrstücks 10 zurückgezogen ist. Die Endoprothese 1 liegt nun auf ihrer ganzen Länge mit einem bestimmten Stützdruck innenseitig an der Gefässwandung 11 an. Die Vorrichtung 2 wird nun vollständig zurückgezogen, wobei die Endoprothese 1 im Gefäss verbleibt. Falls erforderlich, können auf diese Weise hintereinander und miteinander mehrere Endoprothesen plaziert werden.

Wie bereits oben erwähnt, ist es vorgesehen, die Endoprothese 1 unmittelbar vor der Verwendung der Vorrichtung 2 zu montieren. Hierbei wird die Endoprothese 1 in geeigneter Länge von einem längeren Stück abgeschnitten. Bei teilweise zurückgezogenem Aussenkatheter 3 wird dann die Endoprothese 1 von Hand gespannt und in die Vertiefung 14 eingesetzt. Gleichzeitig wird der Aussenkatheter 3 in Gegenrichtung des Pfeiles 22 über die Endoprothese 1 und bis zum Anschlag an der Spitze 7 verschoben. Die Vorrichtung 2 kann nun verwendet werden. Durch eine Abzweigung 25 mit einem Hahn 26 und einer flexiblen Schlauchleitung 27 kann durch das Lumen 15 und eine Bohrung 8 der Spitze 7 im Bedarfsfall ein geeignetes Mittel eingespritzt werden.

## Patentansprüche

1. Vorrichtung zum Implantieren einer selbstexpandierenden Endoprothese in ein Gefäss, mit einem flexiblen länglichen Aussenkatheter (3) mit einem distalen Ende (5) und einem proximalen Ende und mit einem koaxial zum Aussenkatheter (3) angeordneten flexiblen länglichen Innenkatheter (6), der an einem distalen Ende eine Spitze (7) und proximal dieser am Umfang eine Vertiefung (14) zur Aufnahme der Endoprothese (1) aufweist, wobei zur Fixierung der Endoprothese (1) der Aussenkatheter (3) in seiner Längsrichtung über die Vertiefung (14) schiebbar und zur Freigabe der Endoprothese (1) ins Gefäss (11) zurückziehbar ist, dadurch gekennzeichnet, dass die Vertiefung (14) zur Aufnahme der Endoprothese (1) durch ein separates Zwischenstück (9,12) des Innenkatheters (6) gebildet ist.

2. Vorrichtung nach Anspruch 1, dadurch gekennzeichnet, dass das Zwischenstück verstärkt ist.

3. Vorrichtung nach Anspruch 1 oder 2, dadurch gekennzeichnet, dass das Zwischenstück (9,12) aus einem Verbundwerkstoff hergestellt ist.

4. Vorrichtung nach Anspruch 1 oder 2, dadurch gekennzeichnet, dass das Zwischenstück (9) mittels Fasern, insbesondere Glasfasern verstärkt ist.

5. Vorrichtung nach Anspruch 4, dadurch gekennzeichnet, dass die Fasern in ein schlauchförmiges Netz verwoben sind.

6. Vorrichtung nach Anspruch 1 oder 2, dadurch gekennzeichnet, dass das Zwischenstück (9,12) wenigstens teilweise aus Metall hergestellt ist.

7. Vorrichtung nach einem der Ansprüche 1 bis 6, dadurch gekennzeichnet, dass das Zwischenstück (9,12) im wesentlichen die Länge der Vertiefung (14) aufweist.

8. Vorrichtung nach einem der Ansprüche 1 bis 7, dadurch gekennzeichnet, dass das Zwischenstück (9,12) hohlzylindrisch ausgebildet ist.

9. Vorrichtung nach einem der Ansprüche 1 bis 8, dadurch gekennzeichnet, dass das Zwischenstück (9,12) einen inneren Durchgang (8) mit einem Innendurchmesser (E) aufweist, der kleiner ist als der Innendurchmesser (D) eines proximal anschliessenden Rohrstücks (10) des Innenkatheters (6).

10. Vorrichtung nach einem der Ansprüche 1 bis 9, dadurch gekennzeichnet, dass das Zwischenstück (9,12) mit dem einen Ende in eine Ausnehmung (8a) der Spitze (7) eingesetzt und in dieser Ausnehmung (8a) beispielsweise mittels eines Klebstoffes (20) befestigt ist.

11. Vorrichtung nach einem der Ansprüche 1 bis 10, dadurch gekennzeichnet, dass das Zwischenstück (9,12) in eine Ausnehmung (10a) eines proximal anschliessenden Rohrstücks (10) des Innenkatheters (6) eingesetzt und in dieser Ausnehmung (10a) beispielsweise mittels eines Klebstoffes befestigt ist.

12. Vorrichtung nach einem der Ansprüche 1 bis 11, gekennzeichnet durch zwei im Abstand zueinander auf das Zwischenstück (9,12) aufgesetzte Markierringe (21), die auf der Aussenseite des Zwischenstücks (9,12) radial vorstehen und die eine in die Vertiefung (14) eingesetzte Endoprothese (1) gegen eine Verschiebung in Längsrichtung distal bzw. proximal fixieren.

13. Vorrichtung nach einem der Ansprüche 1 bis 12, dadurch gekennzeichnet, dass das Zwischenstück (9,12) und ein proximal an dieses angeschlossene Rohrstück (10) des Innenkatheters (6) Abschnitte separat hergestellter Rohre sind.

14. Vorrichtung nach Anspruch 13, dadurch gekennzeichnet, dass das Zwischenstück (9,12) einen äusseren Durchmesser (F) von etwa 2,3 mm und das proximal angeschlossene Rohrstück (10) einen äusseren Durchmesser von etwa 4,5 mm aufweisen.

15. Vorrichtung nach Anspruch 13 oder 14, dadurch gekennzeichnet, dass das Zwischenstück (9,12) einen Innendurchmesser (E) von etwa 1,6 mm und das proximal anschliessende Rohrstück (10) einen Innendurchmesser von etwa 1,8 mm aufweisen.

16. Vorrichtung nach einem der Ansprüche 13 bis 15, dadurch gekennzeichnet, dass das proximal angeschlossene Rohrstück (10) und/oder die Spitze (7) aus Polyurethan hergestellt sind.

## Claims

1. A device for implanting a self-expanding endoprothesis into a vessel comprising a flexible elongated external catheter (3) with a distal end (5) and a proximal end, and a flexible elongated internal catheter (6) arranged coaxially to the external catheter (3), said internal catheter having a point (7) at a distal end, and proximally to said point, a circumferential recess (14) for receiving the endoprosthesis, the external catheter (3) being movable in its longitudinal direction over the recess (14) in order to secure the endoprosthesis (1) and being retractable into the vessel (11) in order to release the endoprosthesis (1), characterised in that the recess (14) for receiving the endoprosthesis is formed by a separate intermediate piece (9,12) of the internal catheter (6).

2. A device according to claim 1, characterised in that the intermediate piece is reinforced.

3. A device according to claim 1 or 2, characterised in that the intermediate piece (9,12) is made of a composite material.

4. A device according to claim 1 or 2, characterised in that the intermediate piece (9) is reinforced by means of fibres, more particularly glass fibres.

5. A device according to claim 4, characterised in that the fibres are woven into a lattice in the form of a hose.

6. A device according to claim 1 or 2, characterised in that the intermediate piece (9,12) is made at least partly of metal.

7. A device according to one of claims 1 to 6, characterised in that the intermediate piece (9,12) is substantially of the length of the recess (14).

8. A device according to one of claims 1 to 7, characterised in that the intermediate piece (9,12) is in the form of a hollow cylinder.

9. A device according to one of claims 1 to 8, characterised in that the intermediate piece (9,12) has an inner passage (8) with an internal diameter (E) which is smaller than the internal diameter (D) of a proximally adjoining tube section (10) of the internal catheter (6).

10. A device according to one of claims 1 to 9, characterised in that the intermediate piece (9,12) has one end inserted into a recess (8a) of the point (7) and secured to said recess (8a), for example by means of adhesive (20).

11. A device according to one of claims 1 to 10, characterised in that the intermediate piece (9,12) is inserted into a recess (10a) of a proximally adjoining tube section (10) of the internal catheter (6), and is secured to said recess (10a), for example by means of adhesive.

12. A device according to one of claims 1 to 11, characterised by two marking rings (21) placed on the intermediate piece (9,12) spaced from one another, said marking rings projecting radially from the outside of the intermediate piece (9,12) and securing an endoprosthesis (1) inserted into the recess (14) distally and proximally against a displacement in the longitudinal direction.

13. A device according to one of claims 1 to 12, characterised in that the intermediate piece (9,12) and a tube section (10) of the internal catheter (6), said tube section being connected proximally to the intermediate piece, are portions of separately produced tubes.

14. A device according to claim 13, characterised in that the intermediate piece (9,12) has an external diameter (F) of about 2.3 mm and that the tube section (10) connected proximally has an external diameter of about 4.5 mm.

15. A device according to claim 13 or 14, characterised in that the intermediate piece (9,12) has an internal diameter (E) of about 1.6 mm and the proximally adjoining tube section (10) has an internal diameter of about 1.8 mm.

16. A device according to one of claims 13 to 15, characterised in that the tube section (10) connected proximally and/or the point (7) are made of polyurethane.

## Revendications

1. Dispositif pour l'implantation d'une endoprothèse à autoexpansion dans un vaisseau, comprenant un cathéter externe longitudinal et flexible (3) avec une extrémité distale (5) et une extrémité proximale et un cathéter interne longitudinal et flexible (6) disposé coaxialement dans le cathéter externe (3), qui présente à une extrémité distale une pointe (7) et sur le côté proximal de cette dernière et sur sa périphérie une cavité (14) destinée au logement d'une endoprothèse (1), le cathéter externe (3) pouvant être coulissé dans sa direction longitudinale au-dessus de la cavité (14) pour maintenir l'endoprothèse et pouvant être ramené en arrière pour libérer l'endoprothèse (1) dans le vaisseau (11), caractérisé en ce que la cavité (14) destinée au logement de l'endoprothèse (1) est constitué par un élément intermédiaire séparé (9, 12) du cathéter interne (6).

2. Dispositif selon la revendication 1, caractérisé en ce que l'élément intermédiaire est renforcé.

3. Dispositif selon la revendication 1 ou 2, caractérisé en ce que l'élément intermédiaire (9, 12) est réalisé en un matériau stratifié.

4. Dispositif selon la revendication 1 ou 2, caractérisé en ce que l'élément intermédiaire (9) est renforcé par des fibres et notamment des fibres de verre.

5. Dispositif selon la revendication 4, caractérisé en ce que les fibres sont tressées sous forme d'un filet en forme de tuyau flexible.

6. Dispositif selon la revendication 1 ou 2, caractérisé en ce que l'élément intermédiaire (9, 12) est réalisé au moins en partie en métal.

7. Dispositif selon l'une quelconque des revendications 1 à 6, caractérisé en ce que l'élément intermédiaire (9, 12) a sensiblement la même longueur que la cavité (14).

8. Dispositif selon l'une quelconque des revendications 1 à 7, caractérisé en ce que l'élément intermédiaire (9, 12) est de forme cylindrique et creuse.

9. Dispositif selon l'une quelconque des revendications 1 à 8, caractérisé en ce que l'élément intermédiaire (9, 12) comporte un passage interne (8) dont le diamètre interne (E) est plus petit que le diamètre interne (D) d'un élément de tube (10) du cathéter interne (6) qui se raccorde à son côté proximal.

10. Dispositif selon l'une quelconque des revendications 1 à 9, caractérisé en ce que l'élément intermédiaire (9, 12) est introduit par une extrémité dans un évidement (8a) de la pointe (7) et est fixé dans cet évidement (8a) par exemple au moyen d'une colle (20).

11. Dispositif selon l'une quelconque des revendications 1 à 10, caractérisé en ce que l'élément intermédiaire (9, 12) est introduit dans un évidement (10a) d'un élément de tube (10) du cathéter interne (6) qui s'y raccorde sur le côté proximal et est fixé dans cet évidement (10a) par exemple au moyen d'une colle.

12. Dispositif selon l'une quelconque des revendications 1 à 11, caractérisé en ce que deux anneaux de délimitation (21) sont disposés à une certaine distance l'un de l'autre sur l'élément intermédiaire (9, 12), qui font saillie radialement sur le côté externe de l'élément intermédiaire (9, 12) et qui maintiennent dans une position distale ou proximale une endoprothèse (1) introduite dans la cavité (14) en l'empêchant de se déplacer en direction longitudinale.

13. Dispositif selon l'une quelconque des revendications 1 à 12, caractérisé en ce que l'élément intermédiaire (9, 12) et un élément de tube (10) du cathéter interne (6) qui s'y raccorde sur le côté proximal sont constitués par des sections de tubes réalisés séparément.

14. Dispositif selon la revendication 13, caractérisé en ce que l'élément intermédiaire (9, 12) a un diamètre externe (L) d'environ 2,3 mm, et l'élément de tube (10) qui lui est raccordé sur son côté proximal a un diamètre externe d'environ 4,5 mm.

15. Dispositif selon la revendication 13 ou 14, caractérisé en ce que l'élément intermédiaire (9, 12) a un diamètre interne (E) d'environ 1,6 mm et l'élément de tube (10) qui s'y raccorde sur son côté proximal a un diamètre interne d'environ 1,8 mm.

16. Dispositif selon l'une quelconque des revendications 13 à 15, caractérisé en ce que l'élément de tube (10) qui est raccordé sur le côté proximal et/ou la pointe (7) sont réalisés en polyuréthane.
